(19) Europäisches Patentamt
European Patent Office
Office européen des brevets

(11) Veröffentlichungsnummer: **0 300 323 B1**

(12) **EUROPÄISCHE PATENTSCHRIFT**

(45) Veröffentlichungstag der Patentschrift: **04.03.92**

(51) Int. Cl.[5]: **C07C 213/02**, C07C 215/08

(21) Anmeldenummer: **88111079.5**

(22) Anmeldetag: **12.07.88**

(54) **Verfahren zur Herstellung von aliphatischen N,N-dialkylsubstituierten Aminoalkoholen.**

(30) Priorität: **22.07.87 DE 3724239**

(43) Veröffentlichungstag der Anmeldung:
**25.01.89 Patentblatt 89/04**

(45) Bekanntmachung des Hinweises auf die Patenterteilung:
**04.03.92 Patentblatt 92/10**

(84) Benannte Vertragsstaaten:
**BE DE ES FR GB IT**

(56) Entgegenhaltungen:
**EP-A- 0 006 454**
**EP-A- 0 070 512**
**EP-A- 0 147 990**
**DE-A- 611 283**
**FR-A- 2 351 088**
**US-A- 3 270 059**
**US-A- 4 680 393**

(73) Patentinhaber: **BASF Aktiengesellschaft**
**Carl-Bosch-Strasse 38**
**W-6700 Ludwigshafen(DE)**

(72) Erfinder: **Fischer, Roman, Dr.**
**Deidesheimer Strasse 1**
**W-6704 Mutterstadt(DE)**
Erfinder: **Mercker, Hans Jochen, Dr.**
**Schwabenstrasse 14**
**W-6800 Mannheim 61(DE)**
Erfinder: **Mueller, Herbert, Dr.**
**Carostrasse 53**
**W-6710 Frankenthal(DE)**

EP 0 300 323 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von aliphatischen N,N-dialkylsubstituierten Aminoalkoholen der allgemeinen Formel I

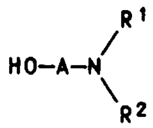

(I)

in der A ein aliphatisches Brückenglied mit mindestens 2 C-Atomen bezeichnet und $R^1$ und $R^2$ für $C_1$-$C_6$-Alkylgruppen stehen, die zu einem 5-oder 6-gliedrigen Ring verbunden sein können, durch Umsetzung eines Diols II

HO-A-OH (II)

mit einem sekundären Amin III

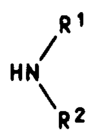

(III)

in Gegenwart eines Hydrierungs-/Dehydrierungskatalysators, sowie in Gegenwart von Wasserstoff und einer basischen Alkalimetall- oder Erdalkalimetallverbindung in flüssiger Phase bei erhöhter Temperatur und unter erhöhtem Druck.

Dieses Verfahrensprinzip zur Herstellung tertiärer Amine aus Alkoholen und sekundären Aminen unter hydrierenden Bedingungen

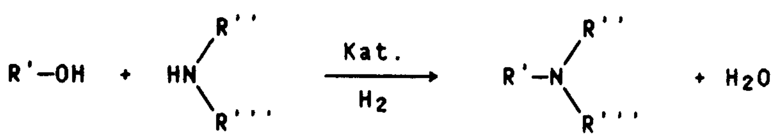


R'-R''' = organische Reste

ist allgemein bekannt, erfordert aber zur Erzielung befriedigender Ergebnisse im Einzelfall wegen zahlreicher Nebenreaktionen je nach den Einsatzstoffen spezielle Reaktionsbedingungen.

So wird in der DE-C 26 25 196 empfohlen, die Umsetzung von ein- oder mehrwertigen Alkoholen und sekundären Aminen in flüssiger Phase in Gegenwart von Hydrierungs-/Dehydrierungskatalysatoren und von basischen Alkali- oder Erdalkalimetallverbindungen vorzunehmen und das dabei entstehende Wasser laufend aus dem Reaktionsgemisch zu entfernen. So sehr sich diese Arbeitsweise allgemein bewährt, so läßt sie doch für den Fall der Herstellung der Aminoalkohole I wegen mangelnden Selektivitäten zu wünschen übrig.

Ähnlich verhält es sich mit dem Verfahren der EP-B 70 512, welches sich von dem Verfahren der DE-C 26 25 196 dadurch unterscheidet, daß man einen Katalysator verwendet, wie er unter den Reaktionsbedingungen aus Kupferformiat von selbst entsteht. Auch hiernach gelingt die Umsetzung von Diolen zu Aminoalkoholen weniger gut als von Monoalkoholen zu tertiären Aminen.

Ferner ist es aus der DE-A 28 24 908 bekannt, Diole des Typs II in Gegenwart von Wasserstoff und u.a. Kupferkatalysatoren in flüssiger Phase mit sekundären Aminen in einer zweistufigen Reaktion unter Entfernung des Reaktionswassers in die entsprechenden bis-tertiären Diamine zu überführen, wobei die N,N-disubstituierten Aminoalkohole des Typs I als Nebenprodukte anfallen.

Der Erfindung lag die Aufgabe zugrunde, die besonders für organische Synthesen wichtigen N,N-dialkylsubstituierten Aminoalkohole I in höheren Ausbeuten und Selektivitäten zugänglich zu machen als

bisher.

Demgemäß wurde ein Verfahren zur Herstellung von aliphatischen N,N-dialkylsubstituierten Aminoalkoholen der allgemeinen Formel I

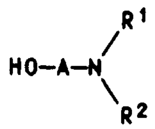 (I)

in der A ein aliphatisches Brückenglied mit mindestens 2 C-Atomen bezeichnet und $R^1$ und $R^2$ für $C_1$-$C_6$-Alkylgruppen stehen, die zu einem 5-oder 6-gliedrigen Ring verbunden sein können, durch Umsetzung eines Diols II

HO-A-OH (II)

mit einem sekundären Amin III

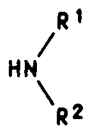 (III)

in Gegenwart eines Hydrierungs-/Dehydrierungskatalysators, sowie in Gegenwart von Wasserstoff und einer basischen Alkalimetall- oder Erdalkalimetallverbindung in flüssiger Phase bei erhöhter Temperatur und unter erhöhtem Druck gefunden, welches dadurch gekennzeichnet ist, daß man die Umsetzung

a) bei 150 bis 250 °C,

b) unter einem Gesamtdruck von 40 bis 250 bar,

c) unter einem Wasserstoffpartialdruck von 2 bis 60 bar,

d) in Gegenwart eines Hydrierungs-/Dehydrierungskatalysators auf Basis von Kupfer sowie

e) in Gegenwart von 1 bis 20 Gew.% Wasser, bezogen auf die Menge des Reaktionsgemischs,

vornimmt.

Als Ausgangsverbindungen II kommen insbesondere bis-primäre Alkandiole in Betracht, in denen A für eine lineare unverzweigte $\alpha,\omega$-Alkylengruppe mit 2 bis 8 C-Atomen steht, also z.B. Ethylenglycol, Propan-1,3-diol, Butan-1,4-diol, Pentan-1,5-diol und Hexan-1,6-diol. Von besonderer Bedeutung sind außerdem Etherdiole wie Diethylenglycol (3-Oxapentan-1,5-diol), Triethylenglycol (3,6-Dioxaoctan-1,8-diol) und 1,3-Dipropylenglycol (4-Oxaheptan-1,7-diol).

Allgemein eignen sich als Diole II solche mit Vorzugsweise primären Hydroxylgruppen, wogegen Diole mit sekundärer und tertiärer Alkoholfunktion von untergeordneter Bedeutung sind. Ferner ist das Brückenglied vorzugsweise eine lineare Alkylengruppe, die durch Ethersauerstoff unterbrochen sein kann, jedoch kann es auch Methyl-, Ethyl-, Methoxy- und Ethoxygruppen als Substituenten tragen. Die Gesamtzahl der C-Atome in A beträgt vorzugsweise bis zu 12, besonders bis zu 6.

Unter den sekundären Dialkylaminen sind an erster Stelle solche mit bis zu 6 C-Atomen zu nennen, z.B. Methylpropylamin, Methylbutylamin, Ethylbutylamin, Di-n-propylamin und Di-iso-propylamin sowie vor allem Dimethylamin, Diethylamin und Methylethylamin. Die Alkylgruppen können auch zu einem 5- oder 6-gliedrigen heterocyclischen Ring verbunden sein; Beispiele derartiger Amine sind Pyrrolidin, Piperidin und deren Methylhomologe.

Das Molverhältnis von Diol zu Amin beträgt vorzugsweise mindestens 1:1, wobei man besonders gute Ergebnisse mit Molverhältnissen zwischen 2:1 und 5:1 erzielt. Größere Verhältnisse als 10:1 bringen im allgemeinen keine wirtschaftlichen Vorteile mehr.

Hydrierungs-/Dehydrierungskatalysatoren auf Basis von Kupfer sind solche, deren katalytische Aktivität hauptsächlich auf Kupfer zurückzuführen ist und deren aktive Masse demgemäß zu mindestens 80 Gew.% aus diesem Metall besteht.

Man kann das Kupfer in Form von Pulver, Spänen oder als Skelettkatalysator (Raney-Kupfer) einsetzen, jedoch empfiehlt es sich, die Katalysatoren durch Reduktion von Kupferverbindungen wie Kupferacetat, Kupferpropionat und Kupferoxid herzustellen, wobei die Reduktion in situ erfolgen kann.

Sehr gute Ergebnisse erzielt man beispielsweise mit einem Katalysator gemäß der EP-B 70 512, der

sich unter den Reaktionsbedingungen aus Kupferformiat bildet.

Ebenfalls hervorragend geeignete Katalysatoren sind solche, die man nach der DE-A 24 45 303 gewinnt, indem man eine ein Kupfersalz und ein Aluminiumsalz, z.B. Kupfernitrat und Aluminiumnitrat im Atomverhältnis Cu:Al von etwa 0,2:1 bis 1:1, enthaltende wäßrige Lösung mit einem Alkalimetallcarbonat versetzt und das hierbei anfallende basische Cu/Al-Mischcarbonat zu Katalysatorteilchen wie Kugeln oder Strängen formt und anschließend trocknet. Diese Teilchen bilden unter Reaktionsbedingungen ein poröses Gerüst aus Aluminiumoxiden und -oxidhydraten mit einer entsprechend großen Oberflächenschicht aus metallischem Kupfer.

Weiterhin kann man Cu-haltige Trägerkatalysatoren verwenden, die man durch Tränken von Trägermaterialien wie Bimsstein, Diatomeenerde, Kieselgel oder Aluminiumoxid mit Cu-Salz-Lösungen und anschließende reduzierende Behandlung erhält.

Selbstverständlich kann man die Aktivierung der Katalysatoren, d.h. die Reduktion des gebundenen Kupfers zum metallischen Kupfer mit Wasserstoff oder anderen reduzierenden Substanzen wie Alkoholen auch getrennt von der erfindungsgemäßen Reaktion vornehmen.

Bei der diskontinuierlichen Betriebsweise verwendet man die Katalysatoren vorzugsweise in Form feinteiliger Suspensionen, wogegen sich im kontinuierlichen Verfahren Festbettkontakte empfehlen.

Die Menge der Katalysatoren entspricht beim diskontinuierlichen Verfahren vorzugsweise 10 bis 150, besonders 20 bis 70 g Kupfer pro kg Reaktionsgemisch, und für den kontinuierlichen Betrieb liegt sie vorzugsweise im Bereich von 100 bis 500, besonders 200 bis 400 g Cu pro kg Reaktionsgemisch pro Stunde.

Neben der Hauptkomponente Kupfer können die Katalysatoren auch noch untergeordnete Mengen anderer Bestandteile, z.B. Cobalt und Nickel enthalten.

Als basische Alkali- und Erdalkalimetallverbindungen (Mineralbasen), durch deren Anwesenheit die unerwünschten Umalkylierungsreaktionen zurückgedrängt werden, kommen beliebige Verbindungen dieser Art in Betracht, jedoch empfiehlt sich aus wirtschaftlichen Gründen die Verwendung von Natriumhydroxid, Kaliumhydroxid, Natriumcarbonat, Calciumoxid und Calciumhydroxid. Wesentlich ist nur, daß sich aus den Mineralbasen mit Wasser $OH^-$-Ionen bilden, weshalb man sie vorzugsweise in Form wäßriger Lösungen einsetzt. Die basischen Verbindungen sollen im übrigen in freier Form im Reaktionsgemisch vorliegen, d.h. unabhängig von eventuellen basischen Komponenten im Kupferkatalysator. Die Menge dieser basischen Verbindungen beträgt vorzugsweise 0,005 bis 0,2, besonders 0,02 bis 0,1 moleq. pro kg des Reaktionsgemisches.

Unter den Merkmalen der erfindungsgemäßen Kombination ist der Mitverwendung von Wasser besondere Bedeutung beizumessen, wodurch die Selektivität des Verfahrens bezüglich der Aminoalkohole I deutlich erhöht wird.

Da sich bei der Reaktion ohnehin Wasser bildet, genügt es, dieses Wasser einfach im Reaktionsgemisch zu belassen und nicht, wie es den herkömmlichen Empfehlungen entsprach, hieraus zu entfernen. Man braucht dann lediglich in der Anfahrphase der Umsetzung etwas Wasser zuzusetzen, und zwar am besten in Form von wäßrigen Lösungen der basischen Alkali-bzw. Erdalkalimetallverbindungen, wie vorstehend bereits erwähnt. Da sich nach etwa 10 %igem Umsatz genügend Wasser gebildet hat, ist der Wasserzusatz zu Beginn der Reaktion jedoch prinzipiell entbehrlich. Innerhalb des definitionsgemäßen Bereichs von 1 bis 20 Gew.% Wasser, bezogen auf die Menge des Reaktionsgemisches, werden Konzentrationen von 5 bis 10 Gew.% bevorzugt. Oberhalb von 20 Gew.% verlangsamt sich die Reaktion zu sehr, so daß sie für technische Zwecke weniger attraktiv wird.

Im Bereich der Reaktionstemperaturen von 150 bis 250 °C erhält man in der Regel die besten Ergebnisse zwischen 170 und 230 °C.

Während der für die Reaktion einzuhaltende Gesamtdruck von 40 bis 250 bar nicht kritisch ist - im allgemeinen arbeitet man zwischen 50 und 150 bar - sollte der Wasserstoffpartialdruck, der im Bereich von 2 bis 60 bar liegt, zweckmäßigerweise nur 5 bis 80 % des Gesamtdrucks betragen.

In apparativer und verfahrenstechnischer Hinsicht bietet das Verfahren keine Besonderheiten. Allgemein empfiehlt es sich für den diskontinuierlichen Betrieb, das Diol II mit dem darin suspendierten Katalysator und der gelösten oder suspendierten Mineralbase und einen Teil des Wassers vorzulegen und das Amin III nach Maßgabe fortschreitender Reaktion bei der Reaktionstemperatur und unter dem gewählten Druck allmählich hinzuzugeben.

Arbeitet man kontinuierlich, so führt man Diol und Amin im gewählten Molverhältnis über ein Kupferkatalysator-Festbett, wobei Mineralbase und Wasser in einer oder beiden dieser Komponenten gelöst bzw. suspendiert sein können.

In beiden Fällen läßt es sich durch Wahl der Verweilzeiten und der übrigen Reaktionsbedingungen unschwer einrichten, daß das Amin III vollständig oder fast vollständig umgesetzt wird, so daß sich die

übliche destillative Aufarbeitung des Reaktionsgemisches besonders einfach gestaltet. Nicht umgesetzte Ausgangsstoffe einschließlich der Mineralbasen können für weitere Ansätze wiederverwendet oder - bei kontinuierlichem Betrieb - in den Reaktor zurückgeführt werden.

Das erfindungsgemäße Verfahren gestattet die einfache und wirtschaftliche Herstellung der Aminoalkohole I in hohen Ausbeuten und Selektivitäten, wobei ein besonderer Vorteil darin liegt, daß nur sehr wenig hochsiedende Substanzen gebildet werden, so daß die Aufarbeitung der Reaktionsgemische entsprechend weniger Schwierigkeiten bereitet. Diese Aminoalkohole dienen als basische Katalysatoren für die Herstellung von Polyurethanen sowie als Zwischenprodukte für die Herstellung von Farbstoffen, Arzneimitteln und Pflanzenschutzmitteln. Besondere Bedeutung hat es für die Herstellung von

N,N-Dimethylaminoethan-2-ol
N,N-Diethylaminoethan-2-ol
N,N-Dimethylaminobutan-4-ol
N,N-Dimethylaminopentan-5-ol
N,N-Dimethylaminohexan-6-ol
N,N-Dimethylamino-3-oxa-pentan-5-ol und
N,N-Diethylaminobutan-4-ol

aus den entsprechenden Diolen II und sekundären Aminen III.

Beispiel 1

Herstellung von N,N-Dimethylamino-3-oxa-pentan-5-ol

Durch ein senkrechtstehendes Reaktionsrohr (3,2 cm Durchmesser und 125 cm Hohe), welches mit einer Schüttung von 700 ml eines unten beschriebenen Kupferkatalysators beschickt war, wurde bei 210 °C, 120 bar Gesamtdruck und 50 bar Wasserstoffpartialdruck von unten nach oben stündlich ein Gemisch aus 318 g (3 mol) Diethylenglycol, 3 g Wasser, 68 g (1,5 mol) Dimethylamin und 0,3 g 50 gew.%ige Natronlauge geleitet, wobei die Menge des Wassers auf etwa 19 g (rd. 5 Gew.%) anstieg.

Die übliche destillative Aufarbeitung des entspannten Reaktionsgemisches lieferte das N,N-Dimethylamino-3-oxa-pentan-5-ol in einer Ausbeute von 67 %; daneben entstanden noch 2 % N,N,N',N' -Tetramethyl-3-oxa-pentan-1,5-diamin und 2 % N-Methylmorpholin, jeweils bezogen auf das eingesetzte Dimethylamin. Der Umsatz an Dimethylamin betrug 71 % und der Anteil hochsiedender Bestandteile im Reaktionsgemisch lag unter 0,5 Gew.%.

Der Katalysator wurde nach Beispiel 1 der DE-B 24 25 303 durch Fällung als Mischcarbonat mit Natriumhydrogencarbonat aus einer wäßrigen Lösung von Kupfernitrat und Aluminiumnitrat (Atomverhältnis Cu:Al = 1:1,2) hergestellt, wonach das Mischcarbonat zu Zylindern von 3 mm Länge und 3 mm Durchmesser geformt und getrocknet wurde. Die Teilchen wurden in das Rohr gefüllt und darin bei 180 bis 200 °C mit 300 ml/h einer 0,01 gew.%igen methanolischen Natriummethylatlösung und unter 50 bar Wasserstoffdruck in die aktivierte, metallisches Kupfer enthaltende Form überführt.

Beispiel 2

Herstellung von N,N-Dimethylaminobutan-4-ol

Unter den Bedingungen von Beispiel 1 wurden stündlich 270 g (3 mol) Butan-1,4-diol, 68 g (1,5 mol) Dimethylamin und 27 mg 30 gew.%ige wäßrige Kalilauge umgesetzt, wobei die Wasserkonzentration auf rd. 6 Gew.% anstieg.

Nach der gaschromatographischen Analyse des entspannten Reaktionsgemisches hatte sich das N,N-Dimethylaminobutan-4-ol bei einem Dimethylumsatz von 70 % in 65 %iger Ausbeute gebildet. Als Nebenprodukte fielen 3 % N,N,N',N'-Tetramethyl-1,4-diaminobutan und 1 % N-Methylpyrrolidin an. Der Anteil höhersiedender Substanzen im Reaktionsgemisch lag unter 0,5 Gew.%.

Beispiel 3

Herstellung von N,N-Diethylaminoethan-2-ol

Analog Beispiel 1 wurden stündlich 186 g (3 mol) Ethylenglycol, 117 g (1,6 mol) Diethylamin und 150 mg 50 gew.%ige wäßrige Natronlauge bei 200 °C, 150 bar Gesamtdruck und 45 bar Wasserstoffpartialdruck miteinander umgesetzt, wobei die Wasserkonzentration auf 6 Gew.% anstieg.

Die destillative Aufarbeitung des entspannten Reaktionsgemisches lieferte das N,N-Diethylaminoethan-2-ol in einer Ausbeute von 50 %. Daneben fielen 4 % N,N,N',N'-Tetraethylethylendiamin an, und der Umsatz an Diethylamin betrug 54 %. Der Anteil an hochsiedenden Nebenprodukten im Reaktionsgemisch belief sich auf etwa 4 Gew.%.

Beispiel 4

Herstellung von N,N-Dimethylamino-3-oxa-pentan-5-ol

Eine Mischung aus 731 g (rd. 7 mol) Diethylenglycol, 156 g (rd. 3,5 mol) Dimethylamin, 5,5 g Calciumhydroxid und 55 g eines metallischen Kupferkatalysators wurden im Laufe von 6 Stunden bei 210 °C, einem Gesamtdruck von 60 bis 80 bar und einem Wasserstoffpartialdruck von 40 bar miteinander umgesetzt, wobei die Wasserkonzentration auf rd. 6 Gew.% anstieg.

Die destillative Aufarbeitung des Reaktionsgemisches lieferte den Aminoalkohol in einer Ausbeute von 68 %; daneben entstanden noch 9 % der Bisaminoverbindung und 11 % N-Methylmorpholin. Der Umsatz an Dimethylamin betrug 89 % und der Hochsiederanteil im Reaktionsgemisch belief sich auf ungefähr 0,3 Gew.%.

Wurde das Reaktionswasser während der Reaktion laufend durch Kreisgasführung und Partialkondensation weitgehend entfernt, sank die Ausbeute am gewünschten Verfahrensprodukt auf 25 %.

Der Katalysator wurde in einer Vorreaktion bei 200 °C durch Reduktion von Kupferformiat mit Wasserstoff in einer Lösung aus Laurylalkohol hergestellt, welche mit Dimethylamin gesättigt war.

**Patentansprüche**

**1.** Verfahren zur Herstellung von aliphatischen N,N-dialkylsubstituierten Aminoalkoholen der allgemeinen Formel I

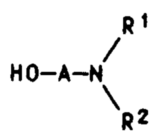

(I)

in der A ein aliphatisches Brückenglied mit mindestens 2 C-Atomen bezeichnet und $R^1$ und $R^2$ für $C_1$-$C_6$-Alkylgruppen stehen, die zu einem 5-oder 6-gliedrigen Ring verbunden sein können, durch Umsetzung eines Diols II

HO-A-OH (II)

mit einem sekundären Amin III

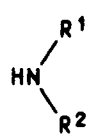

(III)

in Gegenwart eines Hydrierungs-/Dehydrierungskatalysators, sowie in Gegenwart von Wasserstoff und einer basischen Alkalimetall- oder Erdalkalimetallverbindung in flüssiger Phase bei erhöhter Temperatur und unter erhöhtem Druck, dadurch gekennzeichnet, daß man die Umsetzung

a) bei 150 bis 250 °C,
b) unter einem Gesamtdruck von 40 bis 250 bar,
c) unter einem Wasserstoffpartialdruck von 2 bis 60 bar,
d) in Gegenwart eines Hydrierungs-/Dehydrierungskatalysators auf Basis von Kupfer sowie
e) in Gegenwart von 1 bis 20 Gew.% Wasser, bezogen auf die Menge des Reaktionsgemischs,

vornimmt.

2. Verfahren nach Anspruch 1, dadurch gekennzeichnet, daß man es auf die Umsetzung von linearen α,ω-Alkandiolen mit 2 bis 8 C-Atomen anwendet, wobei bis zu 3 Methylengruppen durch Sauerstoff in Etherfunktion ersetzt sein können.

3. Verfahren nach den Ansprüchen 1 und 2, dadurch gekennzeichnet, daß man es auf die Umsetzung von Dimethylamin und Diethylamin anwendet.

4. Verfahren nach den Ansprüchen 1 bis 3, dadurch gekennzeichnet, daß man als Kupferkatalysatoren solche verwendet, die durch Reduktion von Cu/Al-Mischcarbonaten, die ihrerseits durch gemeinsame Fällung aus wäßrigen, Cu- und Al-Salze enthaltenden Lösungen mit Alkalimetallcarbonaten hergestellt wurden, erhältlich sind.

5. Verfahren nach den Ansprüchen 1 bis 3, dadurch gekennzeichnet, daß man als Kupferkatalysatoren solche verwendet, die durch Reduktion von Kupferformiat erhältlich sind.

**Claims**

1. A process for the preparation of aliphatic N,N-dialkyl-substituted aminoalcohols of the formula I

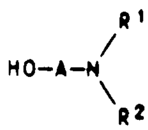

(I)

where A is an aliphatic bridge having at least 2 carbon atoms, and $R^1$ and $R^2$ are $C_1$-$C_6$-alkyl radicals, which may be connected to form a 5- or 6-membered ring, by reacting a diol II

HO-A-OH (II)

with a secondary amine III

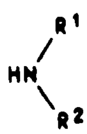

(III)

in the presence of a hydrogenation/dehydrogenation catalyst, and in the presence of hydrogen and a basic alkali metal compound or alkaline earth metal compound, in the liquid phase at elevated temperature and under elevated pressure, which comprises carrying out the reaction

a) at from 150 to 250 °C,
b) under an overall pressure of from 40 to 250 bar,
c) under a hydrogen partial pressure of from 2 to 60 bar,
d) in the presence of a copper-based hydrogenation/de-hydrogenation catalyst, and
e) in the presence of from 1 to 20% by weight of water, based on the amount of reaction mixture.

2. A process as claimed in claim 1, wherein linear α,ω-alkanediols having 2 to 8 carbon atoms and in which up to 3 methylene groups may be replaced by oxygen in ether functions are reacted.

3. A process as claimed in claim 1 or 2, wherein dimethylamine and diethylamine are reacted.

4. A process as claimed in claim 1 or 2 or 3, wherein the copper catalyst used is obtainable by reducing Cu/Al mixed carbonates which themselves have been prepared by joint precipitation of aqueous solutions containing copper and aluminum salts using alkali metal carbonates.

**5.** A process as claimed in claim 1 or 2 or 3, wherein the copper catalyst used is obtainable by reducing copper formate.

**Revendications**

**1.** Procédé de préparation d'amino-alcools aliphatiques N,N-dialkyl-substitués de formule générale I

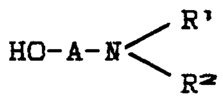

(I)

dans laquelle A désigne un maillon aliphatique de pontage renfermant au moins 2 atomes de carbone, et R' et $R^2$ sont mis pour des groupements alkyle en $C_1$-$C_6$ qui peuvent être reliés en un noyau penta- ou hexagonal, par réaction d'un diol II

HO-A-OH (II)

avec une amine secondaire III

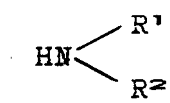

(III)

en présence d'un catalyseur d'hydrogénation/déshydrogénation, ainsi qu'en présence d'hydrogène et d'un composé de métal alcalin ou de métal alcalino-terreux basique, en phase liquide, à température élevée et sous pression élevée, caractérisé en ce qu'on procède à la réaction

a) à une température de 150 à 250 ° C,
b) sous une pression totale de 40 à 250 bar,
c) sous une pression partielle d'hydrogène de 2 à 60 bar,
d) en présence d'un catalyseur d'hydrogénation/déshydrogénation à base de cuivre, ainsi que
e) en présence de 1 à 20% en poids d'eau, par rapport à la quantité du mélange réactionnel.

**2.** Procédé selon la revendication 1, caractérisé en ce qu'on l'applique à la transformation d'$\alpha,\omega$-alcanediols linéaires à 2-8 atomes de carbone, dans lesquels jusqu'à 3 groupements méthylène peuvent être remplacés par de l'oxygène ayant une fonction éther.

**3.** Procédé selon la revendication 1 ou 2, caractérisé en ce qu'on l'applique à la transformation de diméthylamine et de diéthylamine.

**4.** Procédé selon l'une quelconque des revendications 1 à 3, caractérisé en ce qu'on utilise, comme catalyseurs à base de cuivre, des catalyseurs qui peuvent être obtenus par réduction de carbonates mixtes de Cu/Al, lesquels ont été préparés de leur côté par précipitation simultanée, par des carbonates de métaux alcalins, à partir de solutions aqueuses contenant des sels de Cu et Al.

**5.** Procédé selon l'une quelconque des revendications 1 à 3, caractérisé en ce qu'on utilise, comme catalyseurs à base de cuivre, des catalyseurs qui peuvent être obtenus par réduction de formiate de cuivre.